# EUROPEAN PATENT SPECIFICATION

(11) **EP 3 632 453 B1**
(45) Date of publication and mention of the grant of the patent: **04.09.2024**
(21) Application number: 18805833.3
(22) Date of filing: 25.05.2018
(51) Int. Cl.: A61K 35/74, A61K 35/742, A61P 21/00

(54) **MUSCLE QUALITY IMPROVEMENT AGENT**
MITTEL ZUR VERBESSERUNG DER MUSKELQUALITÄT
AGENT D'AMÉLIORATION DE LA QUALITÉ MUSCULAIRE

(30) Priority: 26.05.2017 JP 2017105116
(43) Date of publication of application: 08.04.2020
(73) Proprietor: Japan Eco-Science Co. Ltd., Chiba 260-0034 (JP); Keiyo Plant Engineering Co. Ltd., Chiba 272-0033 (JP); National University Corporation Chiba University, Chiba-shi, Chiba 267-8522 (JP); National University Corporation Kanazawa University, Kanazawa-shi, Ishikawa 920-1192 (JP)
(72) Inventor: KODAMA Hiroaki, Chiba-shi Chiba 263-8522 (JP); SATO Naruki, Chiba-shi Chiba 263-8522 (JP); NISHIUCHI Takumi, Kanazawa-shi Ishikawa 920-1192 (JP); MIYAMOTO Hirokuni, Chiba-shi Chiba 260-0034 (JP); ITO Toshiyuki, Ichikawa-shi Chiba 272-0033 (JP); MORI Kenichi, Chiba-shi Chiba 260-0034 (JP)
(74) Representative: Hauck Patentanwaltspartnerschaft mbB
(86) International application number: PCT/JP2018/020245
(87) International publication number: WO 2018/216818

(56) References cited:
- CN-A- 103 416 585
- JP-A- 2008 113 652
- JP-A- 2009 100 728
- JP-A- 2011 101 597
- JP-A- 2016 216 408
- JP-B2- 5 578 375
- YI-MING CHEN ET AL: "Lactobacillus plantarum TWK10 Supplementation Improves Exercise Performance and Increases Muscle Mass in Mice", NUTRIENTS, vol. 8, no. 4, 7 April 2016 (2016-04-07), pages 205, XP055767589, DOI: 10.3390/nu8040205
- MIRIAM VAN DIJK ET AL: "Improved muscle function and quality after diet intervention with leucine-enriched whey and antioxidants in antioxidant deficient aged mice", ONCOTARGET, vol. 7, no. 14, 5 April 2016 (2016-04-05), pages 17338 - 17355, XP055767676, DOI: 10.18632/oncotarget.7800
- YANG, J. ET AL.: "Effects of chromium-enriched bacillus subtilis KT260179 supplementation on chicken growth performance, plasma lipid parameters, tissue chromium levels, cecal bacterial composition and breast meat quality", LIPIDS IN HEALTH AND DISEASE, vol. 15, no. 1, December 2016 (2016-12-01), pages 188, XP055563498
- MUROYA, SUSUMU: "Basic research on muscle protein troponin T, aiming at improving added value of meat", MEAT SCIENCE, vol. 48, no. 1, 2007, pages 20 - 28, XP009517875
- MAIKEN V. KRÖGER-OHLSEN ET AL: "Free Radicals in Food : Chemistry, Nutrition, and Health Effects", vol. 807, 4 March 2002, AMERICAN CHEMICAL SOCIETY, article KRÖGER-OHLSEN MV ET AL.: "Chapter 10 Pseudoperoxidase activity of myoglobin : Pigment catalyzed formation of radicals in meat systems. ACS SYMPOSIUM SERIES 807 : Free Radicals in Food, Chemistry", pages: 138 - 150, XP055563501

## Description

### TECHNICAL FIELD

The present disclosure relates to a muscle modifier that can be used as a pharmaceutical, food, food additive, feed, and feed additive. The present invention is defined in the appended claims.

### BACKGROUND ART

In recent years, with development of comprehensive biological information analysis technology, so-called omics technology, based on rapid progress of analysis technology and data analysis technology, a relation of intestinal microbiota, that is, intestinal flora, metabolism and physiological function of host animal, and pathology is rapidly becoming clear.

A wide variety of intestinal bacteria inhabit the intestinal tract, interacting with host intestinal cells to form a complex intestinal ecosystem, and maintaining their homeostasis contributes to maintain human health. On the other hand, it has been reported that when the balance is disturbed, it leads to not only intestinal related diseases such as inflammatory bowel disease and colon cancer, but also systemic diseases such as allergies and metabolic diseases (Non Patent Literature 1).

Also, as epidemiological findings, for example, there has been reported a finding that compositions of microbiota in the stools of elderly subjects are grouped according to place of residence and contents of meals, and also the division of the microbiota composition shows significant correlation with determination results of flail, comorbidity, nutritional status, inflammation marker, metabolites in fecal water, and the like (Non Patent Literature 2).

On the other hand, the inventors have already found that, in the disclosure and research that preceded the present disclosure, complex bacteria ATCC PTA-1773 derived from a high-temperature fermentation product found by themselves and Bacillus hisashii NITE BP-863 are respectively orally administered as probiotics, thereby producing an effect of controlling the intestinal microbiota of the administered individual (Non Patent Literature 3) (Patent Literature 1). The complex bacteria ATCC PTA-1773 derived from a high-temperature fermentation product has been internationally deposited with ATCC (American Type Culture Collection, 10801 University Boulevard Manassas, Virginia 20110-2209 USA) on May 1, 2000 (Accession Number: PTA-1773). In addition, the Bacillus hisashii NITE BP-863 has been internationally deposited with National Institute of Technology and Evaluation Patent Microorganisms Depositary (NPMD) (2-5-8 Kazusakamatari, Kisarazu-shi, Chiba, Japan, 292-0818) on Jan. 15, 2010 (Accession No. NITE BP-863).

As a result of administration of thermophilic complex bacteria NITE BP-1051 obtained from the complex bacteria ATCC PTA-1773 derived from a high-temperature fermentation product and Bacillus hisashii NITE BP-863 in the subsequent research and development, the inventors have found as a new finding that, after changes in the intestinal microbiota were caused, physiological changes accompanying muscle modification are caused on the administered individual side. Moreover, the thermophilic complex bacteria NITE BP-1051 has been internationally deposited with National Institute of Technology and Evaluation Patent Microorganisms Depositary (NPMD) (2-5-8 Kazusakamatari, Kisarazu-shi, Chiba, Japan, 292-0818) on Jan. 18, 2011 (Accession No. NITE BP-1051).

The muscle modification here is not limited to changes in muscle mass (weight and volume), as exemplified later, but means that changes at a biochemical/molecular biological level are caused in muscle fibers in muscle tissue and cells, for example, through a regulatory mechanism at an expression level of a gene encoding a muscle protein.

Regarding the effect that the complex bacteria ATCC PTA-1773 derived from a high-temperature fermentation product, and the thermophilic complex bacteria NITE BP-1051 obtained from PTA-1773 and Bacillus hisashii NITE BP-863 cause muscle modification by oral administration, there is no description in which such an effect can be presumed in patents and documents related to the prior art, and in that respect, it can be said that the technology based on the present disclosure is a technology different from the prior art.

Moreover, prior arts which affect the muscle of the administered individual by oral administration of a probiotic microorganism include the followings.

In Patent Literature 2, it is claimed that the muscle mass and the action amount of mice are changed by oral administration of Lactobacillus gasseri.

Also, it has been reported that physical properties, pH, and nutrient composition of broiler muscles are affected by, oral administration of three Bacillus species in Non Patent Literature 4, and oral administration of Bacillus subtilis in Non Patent Literature 5, respectively.

However, all of these prior arts are fundamentally different technologies in that modification of muscle constituent proteins is not targeted, as compared with the muscle modifier of the present disclosure that achieves muscle modification of an administered individual by oral administration.

### CITATION LIST

### PATENT LITERATURE

Patent Literature 1: JP 2016-204355 A
Patent Literature 2: JP 2016-84358 A
Patent Literature 3: JP 2016-216408 A
Patent Literature 4: JP2009100728
Patent Literature 5: JP2011101597
Patent Literature 6: JP5578375B
Patent Literature 7: CN103416585
Patent Literature 8: JP2008113652

### NON PATENT LITERATURE

Non Patent Literature 1: Shingo Fukuda. "Toward understanding and regulation of gut ecosystem by metabologenomics Journal of Japanese Biochemical Society 88.1 (2016): 61-70.
Non Patent Literature 2: Claesson, Marcus J., et al. "Gut microbiota composition correlates with diet and health in the elderly. " Nature 488.7410 (2012): 178-184.
Non Patent Literature 3: The Strategic Core Technology Advancement Program by Ministry of Economy, Trade and Industry FY 2009 "Development of high-functional fermented feed manufacturing technology using waste marine resources and food processing residues"
Non Patent Literature 4: Zhou, Xianjian, et al. "Effects of dietary supplementation of probiotics (Bacillus subtilis, Bacillus licheniformis, and Bacillus natto) on broiler muscle development and meat quality. " Turkish Journal of Veterinary and Animal Sciences 39.2 (2015): 203-210.
Non Patent Literature 5: Abdulla, Nazim Rasul, et al. "Physico-chemical properties of breast muscle in broiler chickens fed probiotics, antibiotics or antibiotic-probiotic mix." Journal of Applied Animal Research 45.1 (2017): 64-70.
Non Patent Literature 6: YANG, J. et al., "Effects of chromium-enriched bacillus subtilis KT260179 supplementation on chicken growth performance, plasma lipid parameters, tissue chromium levels, cecal bacterial composition and breast meat quality", Lipids in Health and Disease, (20161200), vol. 15, no. 1.
Non Patent Literature 7: MIRIAM VAN DIJK ET AL, "Improved muscle function and quality after diet intervention with leucine-enriched whey and antioxidants in antioxidant deficient aged mice", ONCOTARGET, (20160405), vol. 7, no. 14.
Non Patent Literature 8: YI-MING CHEN ET AL, "Lactobacillus plantarum TWK10 Supplementation Improves Exercise Performance and Increases Muscle Mass in Mice", NUTRIENTS, (20160407), vol. 8, no. 4.

### TECHNICAL PROBLEMS

As a premise of the present disclosure, there is a situation in which a problem of, so to speak, "disease reserve", a type of accumulating damage little by little in the daily life, different from "diseases" that are actively treated with drugs or the like, such as chronic fatigue syndrome whose chief complaint and main cause are not clear, and metabolic syndrome as a lifestyle-related disease, has become apparent in modern society.

As an approach to such "non-disease", improvement of physical function and maintenance of health through food, so-called "a balanced diet leads to a healthy body (Ishoku-dougen)" has been emphasized in recent years. Thus, the emergence of technologies and products that scientifically embody Ishoku-dougen and contribute to maintaining and developing the health of society as a whole is awaited.

The present invention, as defined in the claims, contributes to solving the above-described problems through the idea of probiotic microorganisms.

### SOLUTION TO PROBLEMS

The present invention, as defined in the claims, solves the problems with a muscle modifier characterized by causing a qualitative change in muscle fibers of an administered individual by oral administration to a human or animal individual.

The muscle modifier ensures its functionality by containing a fermentation product by microorganisms, or separated or isolated live cells or dead cells, and a product obtained by an operation such as extraction, separation, replication or processing from the fermentation product, live cells or dead cells.

In one embodiment of the present disclosure it is possible to provide a muscle modifier ingestible as live bacteria of Bacillus hisashii NITE BP-863 in an amount equivalent to 10² to 10⁹ cells/kg of host body weight/day, or dead cells that exhibit functions equivalent to the amount of live cells, and as a secondary product obtained by an operation such as extraction, separation, replication or processing from the live cells or dead cells.

In one embodiment of the present disclosure, it is possible to provide a muscle modifier ingestible as a fermentation product obtained by adding all or part of the complex bacteria NITE BP-1051 or all or part of the complex bacteria ATCC PTA-1773 to an appropriate fermentation substrate, and passing through a fermentation process, in an amount equivalent to 0.0001 to 10 g/kg of host body weight/day, or live cells or dead cells that exhibit functions equivalent to the amount of fermentation product, and a secondary product obtained by an operation such as extraction, separation, replication or processing from the fermentation product, live cells or dead cells.

However, the actual application amount of the muscle modifier is desirably determined according to the state of intestinal microbiota for each animal species and individual.

For example, it is possible to confirm changes in an intestinal environment due to the administration of muscle modifier by molecular genetic or analytical chemical verification of the intestinal microbiota. Thus, for the administered individual, it is desirable to determine the optimum dose for an individual or a group of individuals growing in a similar environment with reference to the concentration range described above, taking into account such data and the actual physiological findings.

When it is difficult to conduct such analysis verification, It is desirable to determine a provisional dose with reference to the knowledge in the case of administration to similar subjects, and determine the optimal dose for an individual or a group of individuals growing in a similar environment with reference to the concentration range described above by varying the dose according to the condition of the administered individual after the start of administration, and carefully observing the accompanying improvement in physiological findings.

It has already been shown by the prior art that all of these muscle modifiers are used for oral administration to a human or animal individual, thereby passing through the upper gastrointestinal tract of the administered individual without losing its activity and reaching the intestinal tract to control the internal microbiota.

If the intestinal microbiota of the administered individual changes due to the muscle modifier, then an interaction between a microbial group constituting the intestinal microbiota and intestinal cells via signal molecules changes, and the information is transmitted into a host body, whereby various metabolisms in each tissue in the host body change.

The muscle modifier based on the present disclosure increases, for example, the abundance ratio of genus Lactobacillus, Bifidobacterium or Racnospira among the intestinal microbiota of the administered individual, or the abundance ratio of a specific species of these genera, whereby the interaction between the bacteria and intestinal cells via signal molecules changes, and the information is transmitted into a host body, whereby various metabolisms in each tissue in the host body change.

In addition, the muscle modifier based on the present disclosure decreases, for example, the abundance ratio of genus Clostridium, Streptococcus or Enterococcus among the intestinal microbiota of the administered individual, or the abundance ratio of a specific species of these genera, whereby the interaction between the bacteria and intestinal cells via signal molecules changes, and the information is transmitted into a host body, whereby various metabolisms in each tissue in the host body change.

Due to the overall change in the abundance of bacteria of each genus and each species including the genera Lactobacillus, Clostridium, and Streptococcus by the muscle modifier based on the present disclosure, the interaction between the microbial group constituting the intestinal microbiota and the intestinal cells via the signal molecule changed and the information was transmitted into the host body. As a result, a phenomenon that the content of specific protein in the muscle fibers changes is caused, through an action of a muscle-related protein expression signal or the like in cells constituting the muscle fibers.

Mammalian skeletal muscle fibers are roughly divided into type I (slow muscle) and type II (fast muscle) muscle fibers based on their physiological properties and myosin contained.

The type I muscle fibers are rich in mitochondria and undergo sustained contraction using oxygen.

On the other hand, the type II muscle fibers have relatively few mitochondria and undergo instantaneous contraction by glycolysis. The type II muscle fibers are further subdivided into IIa, Ild/x, and lib muscle fibers, in which the former has slower muscle characteristics.

Troponin T is a very effective marker protein in separating muscle fiber types because it expresses (contains) distinct isoforms different from each other, in fast, slow, and myocardial muscle fibers.

For example, since the muscle modifier provided by the present disclosure shifts the troponin T isoform from fTnT1 type to fTnT3 type in the muscle fibers of the administered animal, the type II muscle fibers can be imparted with slow muscle (IIa-like) characteristics.

Further, it is known that the content of myoglobin in muscle fibers increases as the ratio of the type I muscle fibers and the type IIa muscle fibers increases, and, for example, the muscle modifier provided by the present disclosure can arbitrarily increase the myoglobin content in the muscle fibers of the administered animal.

As described above, it is extremely important for the muscle modifier based on the present disclosure to shift the muscle fibers of the administered animal to type IIa-like characteristics in terms of maintaining motility in daily life.

As age increases, the type II muscle fibers degenerate. On the other hand, the type I muscle fibers are relatively maintained. Therefore, a decline in motility due to aging becomes apparent not in a relatively slow movement phase like activity in daily life where the type I muscle fibers are mainly used, but in a form of decrease in agility and instantaneousness mainly due to the type II muscle fibers.

Strength training for the elderly is considered important for the purpose of preventing the decline in motility with aging. The strength training for the elderly shows a tendency to muscle hypertrophy in an early stage, but most of these increases in strength are attributed to neural factors.

With continuous training, certain hypertrophy of the type II muscle fibers is observed even in the elderly, but most of the daily physical activities of the elderly are actions depending on the type I muscle fibers, and when the training is intermittent, the type II muscle fibers are assumed to be atrophic again without being used.

### ADVANTAGEOUS EFFECTS

In the muscle modifier based on the present disclosure, when type II muscle fibers are imparted with slow muscle (close to IIa type) characteristics, the type II muscle fibers can be used more effectively even under daily exercise intensity, and bring a positive effect on maintenance of muscle strength in the elderly as a result.

Thus, when the use of the muscle modifier based on the present disclosure brings a positive effect on the maintenance of muscle strength in the elderly, preventive and improving effects on pathological conditions accompanied by muscle atrophy such as sarcopenia, flail, and locomotive syndrome can be expected.

Moreover, the muscle modifier based on the present disclosure shifts fast muscle fibers of the administered animal to type IIa-like characteristics, for example, thereby increasing aerobic motility of livestock animals, contributing to an improvement in metabolism, and bringing an effect of easily producing relatively low fat and reddish meat.

Furthermore, physical characteristics derived from the structure of the muscle fibers change, thereby also affecting texture and suppleness of the meat, and as a result, the quality of the meat can be improved.

### BRIEF DESCRIPTION OF THE DRAWINGS

Fig. 1 is a protein migration pattern by one-dimensional electrophoresis in Example 1 of the present embodiment.
Fig. 2 is a part of a protein migration pattern by two-dimensional electrophoresis in Example 1 of the present embodiment.
Fig. 3 is a part of a protein migration pattern by two-dimensional electrophoresis of Example 2 of the present embodiment.

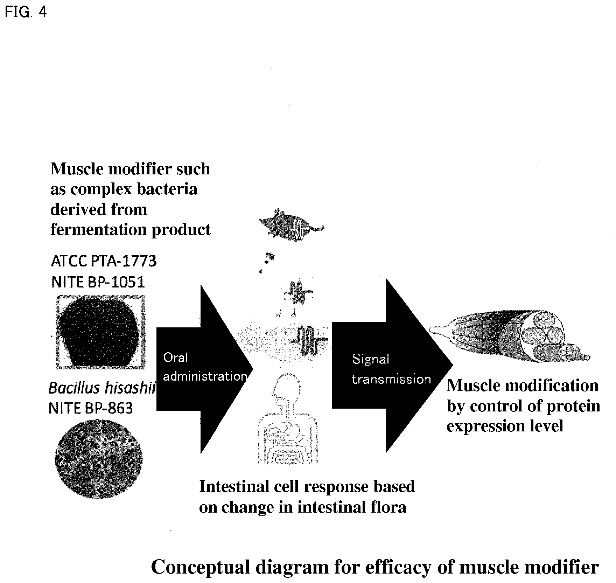

Fig. 4 is a conceptual diagram for an efficacy of a muscle modifier in the present disclosure.

### DESCRIPTION OF EMBODIMENTS

### Example 1

### (Preparation of Fermentation Product Lysate of Thermophilic Bacteria)

A high-temperature fermentation product containing complex bacteria ATCC PTA-1773 was prepared by diluting 100 times by volume with tap water and aeration treatment at 60°C for 10 hours or more.

### (Feeding of Lysate to Pigs)

The prepared lysate containing the complex bacteria ATCC PTA-1773 was mixed into a water supply pipe of a pig breeding facility at a concentration of 0.4% by volume ratio with respect to drinking water, then half of the pigs in the pig breeding facility were fed with drinking water (lysate feeding group), and the other half of the pigs were given drinking water without mixing (control group).

### (Preparation of Pig Thigh Sample)

The thighs of the test pigs were stored at around 0°C for 3 days after slaughter, and then muscle samples were collected and stored frozen at -80°C until analysis.

For extraction of muscle protein, 130 mg of frozen tissue was pulverized with a freeze crusher and then suspended in 650 µL of protein extract, and the protein was further extracted with an ultrasonic crusher.

### (Analysis of Protein Composition by Electrophoresis)

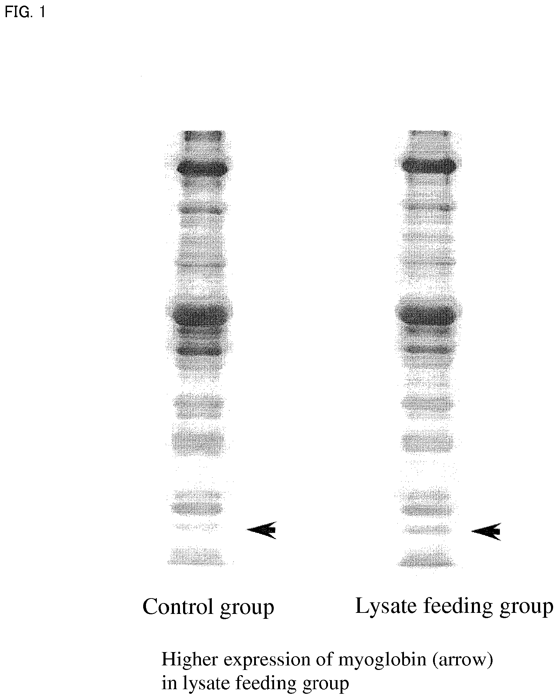

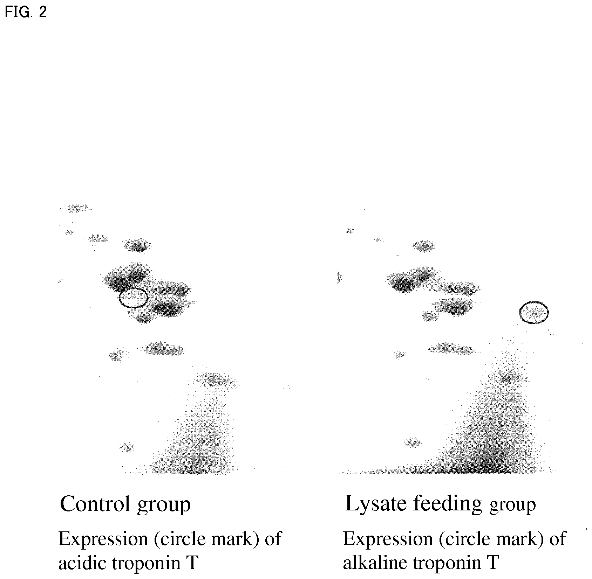

Fig. 1 and Fig. 2 show the results of separating proteins by one-dimensional electrophoresis and two-dimensional electrophoresis using 0.6 µL of the protein extract.

Proteins that showed different mobilities between muscle proteins prepared from the lysate feeding group containing the complex bacteria ATCC PTA-1773 and the control group were analyzed by mass spectrometry to identify the proteins.

Fig. 1 shows a protein migration pattern by one-dimensional electrophoresis. The band indicated by an arrow is myoglobin, which was increased about 1.6 times in the lysate feeding group as compared to the control group.

Myoglobin is a protein that transports oxygen to mitochondria, and an increase in myoglobin increases muscle redness, indicating that there are more type I or type IIa muscle fibers rich in mitochondria.

Fig. 2 shows a part of the protein migration pattern by two-dimensional electrophoresis, in which a particularly different pattern between the lysate feeding group and the control group was obtained. As a result of mass spectrometry, fTnT1 and fTnT2 as acidic troponin T were mainly expressed in the control group, and fTnT3 as alkaline troponin T was mainly expressed in the lysate feeding group.

From the results in Fig. 1 and Fig. 2, by feeding the lysate containing the complex bacteria ATCC PTA-1773, the muscle fibers of pig thigh changed from the type II energy metabolism based on the original glycolytic metabolism to the type IIa-like energy metabolism based on the aerobic metabolism with excellent durability, in which energy metabolism by mitochondria was activated by increase in myoglobin.

### Example 2

### (Preparation of Feed Containing Bacillus hisashii NITE BP-863)

A bait composed of corn and soybean cake meal as main ingredients and formulated and designed to meet the nutrient requirements specified in the Japanese feeding standard was added with Bacillus hisashii NITE BP-863 (3 × 10⁶/g) isolated from fermentation product of thermophilic bacteria at a concentration of 0.01% by weight ratio to prepare a feed.

### (Chicken Breeding)

10-Day male broilers (chunky) were tested and bred for 38 days. The bait prepared in the previous section (BP-863 feeding group) and one having the same formulation but without BP-863 added (control group) were used for ceaseless feeding and free drinking all the time.

### (Preparation of Chicken Superficial Pectoral Muscle)

These broilers were slaughtered and disassembled on day 49 to obtain superficial pectoral muscle samples. Muscle samples were stored frozen at -80°C until analysis. For extraction of muscle protein, 20 mg of frozen tissue was pulverized with a freeze crusher and then suspended in 100 µL of protein extract, and the protein was further extracted with an ultrasonic crusher.

### (Analysis of Protein Composition by Electrophoresis)

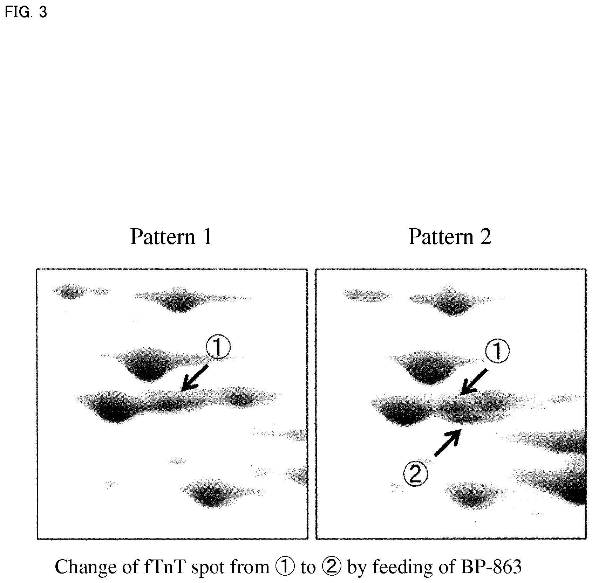

Fig. 3 shows the results of separating proteins by two-dimensional electrophoresis using 1 to 2 µL of the protein extract.

Proteins that showed different mobilities between muscle proteins prepared from the BP-863 feeding group and the control group were analyzed by mass spectrometry to identify the proteins. As a result, the isoform of fast muscle troponin T had changed, and they were classified into the patterns shown in Fig. 3.

All four specimens of the chickens in the control group showed pattern 1, whereas three of the four specimens of the chickens in the BP-863 feeding group showed pattern 2.

This result revealed that the expression of the isoform of fast muscle troponin T can be controlled by feeding the thermophilic bacteria Bacillus hisashii NITE BP-863.

## Claims

1. Non-therapeutic use of at least one of Bacillus hisashii NITE BP-863 as an active ingredient for imparting type II muscle fibers with type IIa-like slow muscle characteristics by shifting the troponin T isoform from fTnT1 type to fTnT3 type in muscle fibers of an administered individual by oral administration to a human or animal individual.

2. A product for use in the treatment of sarcopenia, flail, and locomotive syndrome, comprising at least one of Bacillus hisashii NITE BP-863 as an active ingredient by oral administration to a human or animal individual.

## Patentansprüche

1. Nichttherapeutische Verwendung mindestens eines von Bacillus hisashii NITE BP-863 als einen Wirkstoff zum Versehen von Typ-II-Muskelfasern mit Typ-IIa-ähnlichen Eigenschaften eines langsamen Muskels mittels Verschiebung der Troponin-T-Isoform vom Typ fTnT1 zum Typ fTnT3 in Muskelfasern eines Individuums, dem dieses verabreicht wurde, durch orale Verabreichung an ein menschliches oder tierisches Individuum.

2. Produkt zur Verwendung bei der Behandlung von Sarkopenie, Instabilität und Bewegungssyndrom, das mindestens eines von Bacillus hisashii NITE BP-863 als einen Wirkstoff umfasst, durch orale Verabreichung an ein menschliches oder tierisches Individuum.

## Revendications

1. Utilisation non thérapeutique de l'un au moins parmi Bacillus hisashii NITE BP-863 en tant qu'ingrédient actif destiné à conférer des caractéristiques musculaires lentes du genre IIa à des fibres musculaires de type II par modification de l'isoforme de troponines T du type fTnT1 au type fTnT3 dans des fibres musculaires d'un individu traité par administration orale à un individu humain ou animal.

2. Produit destiné à être utilisé dans le traitement de la sarcopénie, du volet costal, et du syndrome locomoteur, comprenant au moins un Bacillus hisashii NITE BP-863 en tant qu'ingrédient actif par administration orale à un individu humain ou animal.
